# EUROPEAN PATENT APPLICATION

(11) **EP 1 382 344 A1**
(43) Date of publication of application: **21.01.2004**
(21) Application number: 01926078.5
(22) Date of filing: 27.04.2001
(51) Int. Cl.: A61K 31/785, A61P 31/02, A01N 33/12

(54) **BACTERICIDAL DISINFECTANT, ANTIBACTERIAL DRUG, AND ANTIBACTERIAL MATERIAL**

(71) Applicant: Nicca Chemical Co., Ltd., Fukui-shi, Fukui 910-8670 (JP)
(72) Inventor: MIYAMOTO, Hidekazu c/o NICCA CHEMICAL CO., LTD., Fukui-shi, Fukui 910-8670 (JP); MAKINO, Masahiro c/o NICCA CHEMICAL CO., LTD., Fukui-shi, Fukui 910-8670 (JP)
(74) Representative: Gille Hrabal Struck Neidlein Prop Roos
(86) International application number: PCT/JP2001/003753
(87) International publication number: WO 2002/089822

(57) **Abstract**

A sterilizing disinfectant and an antimicrobial agent comprising a cationic polymer represented by general formula [1] and an antimicrobial material using a cationic polymer represented by general formula [1]. 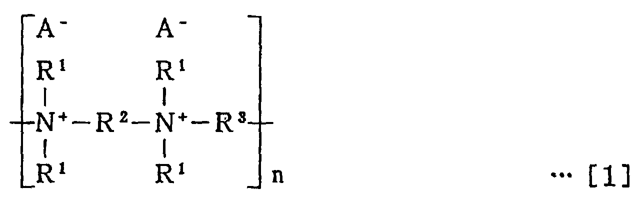 In general formula [1], R¹ represents an alkyl group or a hydroxyalkyl group each having 1 to 4 carbon atoms or an alkenyl group having 2 to 4 carbon atoms, R² represents an alkylene group having 2 to 10 carbon atoms, R³ represents a heteroalkylene group or a heteroalkylene group having hydroxyl group each having 2 to 6 carbon atoms, A represents an anion and n represents a number of 40 to 500.

The sterilizing disinfectant, the antimicrobial agent and the antimicrobial material of the present invention cause no skin disorder such as skin stimulation, rubedo, rough skin and skin sensitization, exhibit effective microbicidal and fungicidal effects in a low concentration and exhibit excellent effects on Pseudomonas aeruginosa.

## Description

### TECHNICAL FIELD

The present invention relates to a sterilizing disinfectant, an antimicrobial agent and an antimicrobial material. More particularly, the present invention relates to a sterilizing disinfectant, an antimicrobial agent and an antimicrobial material which cause no skin disorder such as skin stimulation, rubedo, rough skin and skin sensitization and exhibit effective microbicidal and antimicrobial effects in a low concentration.

### BACKGROUND ART

When conventional sterilizing disinfectants such as sterilizing disinfectants based on surfactants, sterilizing disinfectants based on biguanides and sterilizing disinfectants based on phenols are used, the concentration in the ordinary use is set so as to exhibit the effect of disinfection in a short time such as several ten seconds of contact with microbes. Examples of the sterilizing disinfectants based on surfactants include benzalkonium chloride, benzethonium chloride, didecyldimethylammonium salt, alkyl(C₁₂ to C₁₈)trimethylammonium salts and alkylpolyaminoethylglycine hydrochlorides. These sterilizing disinfectants are used as an aqueous solution or an ethanol solution in a concentration of 0.2% by weight in the ordinary use in medical organizations. It is reported that, when the sterilizing disinfectants based on surfactants are used in the above concentration in the ordinary use, the disinfectants tend to stimulate skin and cause skin disorder such as rough skin, rubedo and skin sensitization. Therefore, from the standpoint of safety, it is desired that the concentration in the use is decreased. However, when the concentration is decreased to a value causing no skin disorder, the effect of disinfection sufficient for achieving the safety cannot be obtained. Moreover, the sterilizing disinfectants based on surfactants have a drawback in that bactericidal effect on Pseudomonas aeruginosa is small.

The use of sterilizing disinfectants causing little skin disorder in a concentration exhibiting a sufficient disinfecting effect has been attempted. For example, cationic polymers based on quaternary ammonium salts which are described in various references [Rembaum, A.: Appli. Polym. Symp., 22, 299 (1973); Vucetic, J. J., Vandjel, V. H. and Janic, M.D.: Glas. Hem. Drus. Beograd, 42, 289 (1977); and Ikeda, T., Yamaguchi, H. and Tazuke, S.: J. Bioact. Comp. Polym., 5, 31 (1990)] exhibit the antimicrobial effect and do not cause the skin disorder such as skin stimulation, rubedo, rough skin and skin sensitization even when these cationic polymers are used in amounts as much as several ten times the amount of the sterilizing disinfectants based on surfactants. However, the cationic polymer has a drawback in that, when the cationic polymer is used in the same concentration as that in the ordinary use of the sterilizing disinfectants based on surfactants, the disinfecting effect of the cationic polymer by a contact with microbes in a short time is inferior to that of the sterilizing disinfectants based on surfactants and, when the cationic polymer is used in a higher concentration so that the sufficient disinfecting effect is exhibited in a short time, an intense adherent feel remains and the use in such a concentration is actually difficult. Under the above circumstances, a sterilizing disinfectant and an antimicrobial agent which cause little skin disorder and exhibit sufficient disinfecting effect in a low concentration have been desired.

The present invention has an object of providing a sterilizing disinfectant, an antimicrobial agent and an antimicrobial material which cause no skin disorder such as skin stimulation, rubedo, rough skin and skin sensitization, exhibit effective microbicidal and antimicrobial effects in a low concentration and exhibit excellent effects on Pseudomonas aeruginosa.

### DISCLOSURE OF THE INVENTION

As the result of extensive studies by the present inventors to overcome the above problems, it was found that a quaternary ammonium polymer having a heteroalkylene structure in the main chain and a high degree of polymerization did not cause skin disorder even when the quaternary ammonium polymer was used in a concentration higher than the concentration in the ordinary use of conventional sterilizing disinfectants, exhibited a disinfecting effect which was the same as or greater than the effect of benzalkonium chloride and exhibited a sufficient disinfecting effect in contact with microbes in a short time when the quaternary ammonium polymer was used in combination with a conventional sterilizing disinfectant based on a surfactant in a concentration causing no skin disorder. The present invention has been completed base on the above knowledge.

The present invention provides:
(1) A sterilizing disinfectant comprising a cationic polymer represented by general formula [1]: 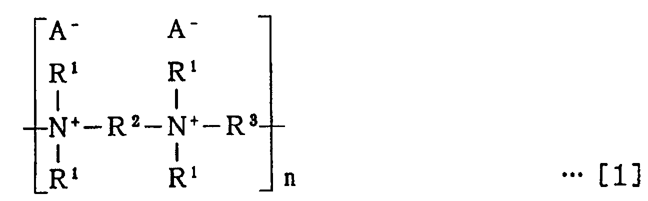 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500;
(2) A sterilizing disinfectant described in (1), wherein the cationic polymer has a weight-average molecular weight of 10,000 to 50,000;
(3) A sterilizing disinfectant described in any one of (1) and (2), which further comprises a sterilizing disinfectant based on a surfactant;
(4) An antimicrobial agent comprising a cationic polymer represented by general formula [1]: 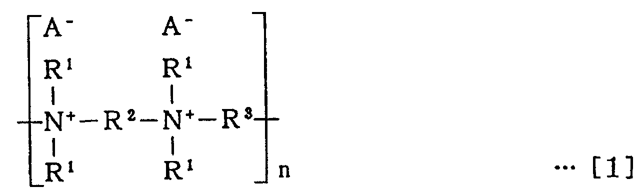 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500;
(5) An antimicrobial material comprising a cationic polymer represented by general formula [1]: 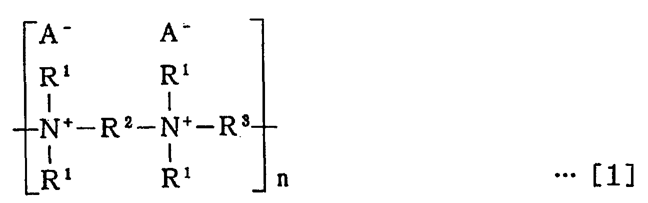 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500; and
(6) An antimicrobial material coated with a cationic polymer represented by general formula [1]: 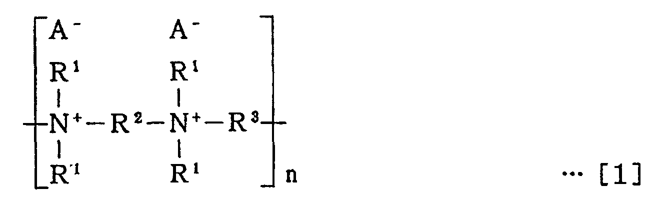 wherein R¹ represents an alkyl group having 1 to 4 carbon atoms, a hydroxy alkyl group having 1 to 4 carbon atoms or an alkenyl group having 2 to 4 carbon atoms, R² represents an alkylene group having 2 to 10 carbon atoms, R³ represents a heteroalkylerie group having 2 to 6 carbon atoms or a heteroalkylene group having hydroxyl group and 2 to 6 carbon atoms, A-represents an anion and n represents a number of 40 to 500.

As preferable embodiments, the present invention also provides:
(7) A sterilizing disinfectant described in (3), wherein the ratio of the amounts of the cationic polymer to the sterilizing disinfectant based on a surfactant is 1,000 or smaller : 1,000; and
(8) A sterilizing disinfectant described in (3), wherein the sterilizing disinfectant based on a surfactant is benzalkonium chloride.

### THE MOST PREFERRED EMBODIMENT TO CARRY OUT THE INVENTION

The sterilizing disinfectant and the antimicrobial agent of the present invention comprise the cationic polymer represented by general formula [1] and the antimicrobial material of the present invention comprises or is coated with the cationic polymer represented by general formula [1]:

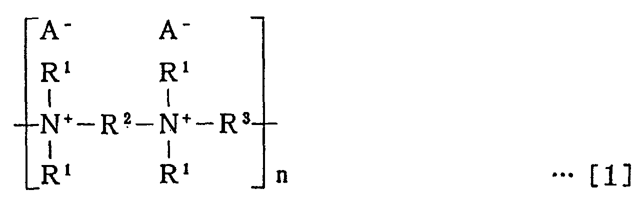

In general formula [1], R¹ represents an alkyl group having 1 to 4 carbon atoms, a hydroxyalkyl group having 1 to 4 carbon atoms or an alkenyl group having 2 to 4 carbon atoms, four R¹ may represent the same group or different groups, R² represents an alkylene group having 2 to 10 carbon atoms, R³ represents a heteroalkylene group having 2 to 6 carbon atoms or a heteroalkylene group having hydroxyl group and 2 to 6 carbon atoms, A- represents an anion and n represents a number of 40 to 500.

Examples of the alkyl group having 1 to 4 carbon atoms include methyl group, ethyl group, n-propyl group, isopropyl group and n-butyl group. Examples of the hydroxyalkyl group having 1 to 4 carbon atoms include hydroxymethyl group, hydroxyethyl group, hydroxypropyl group, 1-methyl-1-hydroxyethyl group and 1-methyl-2-hydroxyethyl group. Examples of the alkenyl group having 2 to 4 carbon atoms include vinyl group and allyl group.

The alkylene group having 2 to 10 carbon atoms is not particularly limited and any of linear alkylene groups and branched alkylene groups can be used. Examples of the alkylene group include ethylene group, propylene group, trimethylene group, tetramethylene group, hexamethylene group, 2-ethylhexamethylene group, octamethylene group and decamethylene group.

The heteroalkylene group having 2 to 6 carbon atoms and the heteroalkylene group having hydroxyl group and 2 to 6 carbon atoms are not particularly limited. It is preferable that the hetero atom is oxygen or sulfur. Examples of the heteroalkylene group include methyleneoxymethylene group, methyleneoxyethylene group, ethyleneoxyethylene group, ethyleneoxymethyleneoxyethylene group, methylenethiomethylene group, methylenethioethylene group and ethylenethioethylene group. Examples of the heteroalkylene group having hydroxyl group include 1-hydroxy-3-oxapentylene group expressed by formula [2] and 1,4-dihydroxy-2-oxabutylene group expressed by formula [3]. The heteroalkylene group having hydroxyl group may have one hydroxyl group or two or more hydroxyl groups.

-CH(OH)CH₂OCH₂CH₂- [2]

-CH(OH)OCH₂CH(OH)- [3]

Examples of the anion represented by A- include anions derived from monobasic or polybasic carboxylic acids such as formic acid, acetic acid, propionic acid, gluconic acid, lactic acid, fumaric acid, maleic acid, adipic acid and tartaric acid; anions of acidic esters of phosphoric acid; anions of esters of alkylsulfuric acids; halogen anions; phosphate anion; sulfate anion; and nitrate anion.

In general formula [1], n represents the average degree of polymerization of the cationic polymer. The average degree of polymerization represented by n can be obtained by measuring the average molecular weight of the cationic polymer and dividing the obtained value by the molecular weight of the repeating unit. When n represents a number smaller than 40, there is the possibility that obtaining the sufficient disinfecting effect is difficult. When n represents a number exceeding 500, viscosity of the cationic polymer increases and there is the possibility that the workability deteriorates.

The sterilizing disinfectant of the present invention is an agent which completely kills microbials of pathogens harmful to the human body and prevents the infection. The antimicrobial agent of the present invention is an agent which prevents growth of microorganisms.

Examples of the cationic polymer represented by general formula [1] include poly[oxyethylene(dimethyliminio)propylene(dimethyliminio)-ethylene dichloride], poly[oxyethylene(dimethyliminio)ethylene(dimethyliminio)ethylene dichloride], poly[oxymethyleneoxyethylene(dimethyliminio)propylene(dimethyliminio)ethylene dichloride] and poly[oxymethyleneoxyethylene(dimethyliminio)ethylene(dimethyliminio)ethylene dichloride].

In the present invention, it is preferable that the weight-average molecular weight of the cationic polymer represented by general formula [1] is in the range of 10,000 to 50,000 and more preferably in the range of 11,000 to 40,000. When the weight-average molecular weight is smaller than 10,000, there is the possibility that obtaining the sufficient disinfecting effect is difficult. When the weight-average molecular weight exceeds 50,000, viscosity of the cationic polymer increases and there is the possibility that workability deteriorates.

The cationic polymer represented by general formula [1] exhibits the effective microbicidal effect and the effective antimicrobial effect in a small concentration and is sufficiently safe to mucous membranes of eyes since little stimulation takes place. Therefore, the cationic polymer can be advantageously used as a liquid agent for the ophthalmology such as an antiseptic for ophthalmic solutions.

The process for producing the cationic polymer represented by general formula [1] is not particularly limited. For example, the cationic polymer can be produced by the reaction of a tetraalkyldiamine represented by general formula [4] and a dichloroheteroalkylene compound represented by general formula [5]:

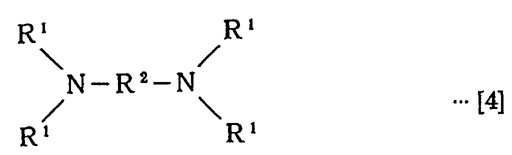

Cl-R³-Cl [5]

In general formula [4], R¹ represents an alkyl group having 1 to 4 carbon atoms, a hydroxyalkyl group having 1 to 4 carbon atoms or an alkenyl group having 2 to 4 carbon atoms, four R¹ may represent the same group or different groups and R² represents an alkylene group having 2 to 10 carbon atoms. In general formula [5], R³ represents a heteroalkylene group having 2 to 6 carbon atoms or a heteroalkylene group having hydroxyl group and 2 to 6 carbon atoms.

Examples of the compound represented by general formula [4] include tetramethylethylenediamine, tetramethylpropylenediamine, tetraethylethylenediamine, tetrakis(2-hydroxyethyl)ethylenediamine and tetraallylethylenediamine. Examples of the compound represented by general formula [5] include di(chloromethyl) ether, bis(2-chloroethyl) ether, di(chloromethyl) formal, bis(2-chloroethyl) formal and bis(2-chloroethyl) sulfide. The compounds represented by general formulae [4] and [5] may be used singly or in combination of two or more.

To the sterilizing disinfectant of the present invention, a sterilizing disinfectant based on a surfactant may be added so that the disinfecting effect in contact in a short time such as several ten seconds is improved. It is preferable that the ratio of the amounts of the sterilizing disinfectant based on a surfactant to the cationic polymer represented by general formula [1] is 1,000 or smaller : 1,000 and more preferably in the range of 5 : 1,995 to 500 : 1,500. When the ratio of the amounts of the sterilizing disinfectant based on a surfactant to the cationic polymer represented by general formula [1] is a value exceeding 1,000 : 1,000, there is the possibility that skin disorder such as skin stimulation, rubedo and rough skin takes place.

Examples of the sterilizing disinfectant based on a surfactant which is comprised in the sterilizing disinfectant of the present invention include sterilizing disinfectants based on cationic surfactants such as benzalkonium chloride, benzethonium chloride, didecyldimethyl ammonium salt and alkyl(C₁₂ to C₁₈)trimethylammonium salts; and sterilizing disinfectants based on amphoteric surfactants such as alkylpolyaminoethylglycine hydrochlorides. The sterilizing disinfectant based on a surfactant may be used singly or in combination of two or more.

The sterilizing disinfectant and the antimicrobial agent of the present invention can be used for microbicidal disinfection of hands, for providing the microbicidal effect to various products by adding to or coating the products and also for obtaining the antimicrobial effect of suppressing growth of microorganisms such as bacteria, fungi and protozoa. Examples of the antimicrobial material of the present invention include materials prepared by mixing the cationic polymer represented by general formula [1] or a solution containing the cationic polymer represented by general formula [1] into natural resins and synthetic resins such as polyester resins, polyurethane resins and acrylic resins and materials in the form of molded articles of the above resins which are coated with the cationic polymer represented by general formula [1] or a solution containing the cationic polymer represented by general formula [1]. Examples of the molded articles include plates, walls and sheets.

### EXAMPLES

The present invention will be described more specifically with reference to examples in the following. However, the present invention is not limited to the examples.

The weight-average molecular weight of a cationic polymer was obtained by the measurement using an aqueous gel permeation chromatograph (GPC) [manufactured by TOSO Co., Ltd.; HLC-8120GPC] and the result was expressed as the corresponding value of polyethylene glycol.

The disinfecting effect was evaluated in accordance with the following methods.
(1) Test microorganisms
   Seven microorganisms, i.e., Staphyrococcus aureus (S. aureus, ATCC 6538P), methicillin-resistant S. aureus (MRSA IID 1677), methicillin-resistant S. aureus (MRSA of a clinically isolated strain), Escherichia coli (E. coli, IFO 3301), Klebsiella pneumoniae (pneumoniae, IFO ATCC 4352), Pseudomonus aeruginosa (P. aeruginosa, IFO 3080) and Serratia marcescens (S. marcescens of a clinically isolated strain), were used for the test.
(2) Preparation of a liquid of a test microorganism
   A test microorganism in an inoculating loop to streak a portion of 8 ml of a culture medium of soybean casein digest (SCD) on the surface and cultured at 37°C for 18 hours. The obtained medium in an amount of 1 ml was diluted with the SCD culture medium to 1/10 of the original concentration. The diluted medium in an amount of 2 ml was brought into contact with 100 ml of the SCD culture medium. The obtained medium was cultured at 37°C for 2 hours except that the medium containing P. aeruginosa was cultured for 4 hours and a liquid of a test microorganism was prepared. The liquid of a test microorganism contained 10⁶ to 10⁸ live microorganisms per 1 ml of the liquid.
(3) Operations
   A series of solutions containing a composition of a sterilizing disinfectant were prepared by dilution with a physiological saline in a manner such that each solution was diluted to one half the concentration to prepare a solution having the next lower concentration. The solutions of a series each in an amount of 10 ml were separately placed into test tubes, treated for disinfection in an autoclave and mixed with a liquid of a test microorganism in an amount of 0.1 ml. After the sterilizing disinfectant and the test microorganism were kept in contact with each other for 1 minute, the obtained liquid for inoculation in an amount of 0.1 ml was added to each of three SCD-LP (lecithin polysolvate) culture media (9 ml, each) which had been disinfected in advance. After the inoculated media were cultured at 37°C for 20 hours, the presence or the absence of turbidity in the media was examined. When the medium was transparent, it was decided that no growth of the microorganism could be recognized and the result for this medium was expressed as -. When the medium was turbid, it was decided that growth of the microorganism could be recognized and the result for this medium was expressed as +. The result exhibited by two or more media among the above three media were used as the result of the test. The minimum concentration of the composition of a sterilizing disinfectant which gave the result of - was used as the minimum bactericidal concentration (MBC).

The test of skin stimulation was conducted in accordance with the following method.

A sterilizing disinfectant in an amount of 0.02 ml was placed on a filter paper and attached to a portion at the inner side of the left upper arm of a person in a manner such that the paper was sealed. The test was conducted with five male persons (A to E) and five female persons (F to J). The primary stimulation was evaluated 30 minutes and 24 hours after the attachment of the paper and the result was classified into four levels. The criterion for the classification was as follows:

| Criterion for the classification | |
|---|---|
| - | negative (no reaction) |
| ± | rubedo alone |
| + | rubedo and humectation or papula |
| ++ | rubedo, edema and marked papula or small blisters |

### Example 1

Water in an amount of 50 g, 25 g of N,N,N',N'-tetramethyl-1,3-propylenediamine and 27 g of bis(2-chloroethyl) ether were mixed together and the reaction was allowed to proceed at 90°C for 25 hours. To the obtained reaction mixture, 19 g of water was added and the concentration of the cationic polymer was adjusted to 40% by weight. The obtained cationic polymer which was poly[oxyethylene(dimethyliminio)propylene(dimethyliminio)ethylene dichloride] had a weight-average molecular weight of 11,500 and the value of n in general formula [1] was 42. The minimum bactericidal concentration of the solution of this cationic polymer was obtained.

### Example 2

Water in an amount of 50 g, 25 g of N,N,N',N'-tetramethyl-1,3-propylenediamine and 27 g of bis(2-chloroethyl) ether were mixed together and the reaction was allowed to proceed at 95°C for 30 hours. To the obtained reaction mixture, 19 g of water was added and the concentration of the cationic polymer was adjusted to 40% by weight. The obtained cationic polymer which was poly[oxyethylene(dimethyliminio)propylene(dimethyliminio)ethylene dichloride] had a weight-average molecular weight of 32,000 and the value of n in general formula [1] was 117. The minimum bactericidal concentration of the solution of this cationic polymer was obtained.

### Example 3

Water in an amount of 50 g, 23 g of N,N,N',N'-tetramethyl-1,3-propylenediamine and 37.5 g of bis(2-chloroethyl) formal were mixed together and the reaction was allowed to proceed at 105°C for 20 hours and then, the unreacted bis(2-chloroethyl) formal was removed by separation into layers. To the resultant mixture, 30.5 g of water was added and the concentration of the cationic polymer was adjusted to 40% by weight. The obtained cationic polymer which was poly[oxymethyleneoxyethylene(dimethyliminio)propylene(dimethyliminio)ethylene dichloride] had a weight-average molecular weight of 13,000 and the value of n in general formula [1] was 43. The minimum bactericidal concentration of the solution of this cationic polymer was obtained.

### Example 4

Water in an amount of 50 g, 25 g of N,N,N',N'-tetramethylhexamethylenediamine and 21 g of bis(2-chloroethyl) ether were mixed together and the reaction was allowed to proceed at 100°C for 48 hours. To the obtained reaction mixture, 19 g of water was added and the concentration of the cationic polymer was adjusted to 40% by weight. The obtained cationic polymer which was poly[oxyethylene(dimethyliminio)-hexamethylene(dimethyliminio)ethylene dichloride] had a weight-average molecular weight of 35,000 and the value of n in general formula [1] was 111. The minimum bactericidal concentration of the solution of this cationic polymer was obtained.

### Example 5

The 40% by weight aqueous solution of the cationic polymer obtained in Example 1 and a 40% by weight aqueous solution of benzalkonium chloride were mixed together in amounts such that the ratio of the amounts was 1,999:1 and a sterilizing disinfectant was prepared. The minimum bactericidal concentration of the prepared sterilizing disinfectant was obtained.

### Example 6

The 40% by weight aqueous solution of the cationic polymer obtained in Example 1 and a 40% by weight aqueous solution of benzalkonium chloride were mixed together in amounts such that the ratio of the amounts was 1,500:500 and a sterilizing disinfectant was prepared. The minimum bactericidal concentration of the prepared sterilizing disinfectant was obtained.

### Example 7

The 40% by weight aqueous solution of the cationic polymer obtained in Example 1 and a 40% by weight aqueous solution of benzalkonium chloride were mixed together in amounts such that the ratio of the amounts was 1,000:1,000 and a sterilizing disinfectant was prepared. The minimum bactericidal concentration of the prepared sterilizing disinfectant was obtained.

### Example 8

The 40% by weight aqueous solution of the cationic polymer obtained in Example 2 and a 40% by weight aqueous solution of benzalkonium chloride were mixed together in amounts such that the ratio of the amounts was 1,999:1 and a sterilizing disinfectant was prepared. The minimum bactericidal concentration of the prepared sterilizing disinfectant was obtained.

### Example 9

The 40% by weight aqueous solution of the cationic polymer obtained in Example 2 and a 40% by weight aqueous solution of benzalkonium chloride were mixed together in amounts such that the ratio of the amounts was 1,500:500 and a sterilizing disinfectant was prepared. The minimum bactericidal concentration of the prepared sterilizing disinfectant was obtained.

### Example 10

The 40% by weight aqueous solution of the cationic polymer obtained in Example 2 and a 40% by weight aqueous solution of benzalkonium chloride were mixed together in amounts such that the ratio of the amounts was 1,000:1,000 and a sterilizing disinfectant was prepared. The minimum bactericidal concentration of the prepared sterilizing disinfectant was obtained.

### Comparative Example 1

Water in an amount of 50 g, 25 g of N,N,N',N'-tetramethyl-1,3-propylenediamine and 27 g of bis(2-chloroethyl) ether were mixed together and the reaction was allowed to proceed at 60°C for 72 hours. To the obtained reaction mixture, 19 g of water was added and the concentration of the cationic polymer was adjusted to 40% by weight. The obtained cationic polymer which was poly[oxyethylene(dimethyliminio)propylene(dimethyliminio)ethylene dichloride] had a weight-average molecular weight of 7,500 and the value of n in general formula [1] was 27. The minimum bactericidal concentration of the solution of this cationic polymer was obtained.

### Comparative Example 2

To a solution obtained by mixing 50 g of water with 15 g of dimethylamine, 32 g of epichlorohydrin was added dropwise under cooling. The reaction was allowed to proceed at 40°C for 15 hours and then at 90°C for 4 hours. To the obtained reaction mixture, 20.5 g of water was added and the concentration of the cationic polymer was adjusted to 40% by weight. The obtained cationic polymer which was poly[2-hydroxyethylene(dimethyliminio)methylene chloride] had a weight-average molecular weight of 10,500 and the average degree of polymerization was 76. The minimum bactericidal concentration of the solution of this cationic polymer was obtained.

### Comparative Example 3

Water in an amount of 50 g, 25 g of tetramethylethylenediamine and 31 g of bis(2-chloroethyl) ether were mixed together and the reaction was allowed to proceed at 70°C for 60 hours. To the obtained reaction mixture, 34 g of water was added and the concentration of the cationic polymer was adjusted to 40% by weight. The obtained cationic polymer which was poly[oxyethylene(dimethyliminio)ethylene(dimethyliminio)ethylene dichloride] had a weight-average molecular weight of 7,500 and the average degree of polymerization was 29. The minimum bactericidal concentration of the solution of this cationic polymer was obtained.

### Comparative Example 4

A 40% by weight aqueous solution of benzalkonium chloride was prepared. The minimum bactericidal concentration of the prepared solution was obtained.

The results in Examples 1 to 10 and in Comparative Examples 1 to 4 are shown in Table 1.

**Table 1**

| Minimum batericidal concentration (ppm) | | | | | | | |
|---|---|---|---|---|---|---|---|
| Test microorganism | S. aureus | MRSA (IID 1677) | MRSA (clinically isolated strain) | E. coli | K. pneumoniae | P. aeruginosa | S. marcescens |
| Example 1 | 125 | <16 | 32 | 250 | 500 | 125 | 500 |
| Example 2 | 125 | <16 | 32 | 250 | 500 | 125 | 500 |
| Example 3 | 125 | <16 | 32 | 500 | 500 | 125 | 500 |
| Example 4 | 125 | <16 | 32 | 250 | 500 | 125 | 500 |
| Example 5 | 32 | <16 | 32 | 64 | 64 | 125 | 64 |
| Example 6 | 32 | <16 | 32 | 64 | 64 | 125 | 64 |
| Example 7 | 32 | <16 | 32 | 64 | 64 | 125 | 64 |
| Example 8 | 32 | <16 | 32 | 64 | 64 | 125 | 64 |
| Example 9 | 32 | <16 | 32 | 64 | 64 | 125 | 64 |
| Example 10 | 32 | <16 | 32 | 64 | 64 | 125 | 64 |
| Comparative Example 1 | 500 | 32 | 64 | 2,000 | 2,000 | 250 | 1,000 |
| Comparative Example 2 | 500 | 125 | 125 | >1,000 | >1,000 | 1,000 | >1,000 |
| Comparative Example 3 | 500 | 16 | 64 | >1,000 | >1,000 | 500 | 1,000 |
| Comparative Example 4 | 32 | 32 | 64 | 64 | 64 | 250 | 64 |

The solutions of the cationic polymers obtained in Examples 1 to 4 had small minimum bactericidal concentrations and exhibited excellent disinfecting effects. In particular, the disinfecting effects on MRSA (IID 1677) and MRSA (a clinically isolated strain) were more excellent than that of benzalkonium chloride of Comparative Example 4, which has heretofore been used as an excellent sterilizing disinfectant. The sterilizing disinfectants of Examples 5 to 10 which contained the solutions of the cationic polymers obtained in Examples 1 and 2 and benzalkonium chloride had the minimum bactericidal concentrations which were the same as or smaller than that of benzalkonium chloride with respect to all types of the test microorganisms and exhibited excellent disinfecting effects. In contrast, the cationic polymers in Comparative Examples 1 and 3 which had the structure represented by general formula [1] but smaller molecular weights and the cationic polymers in Comparative Example 2 which had a high molecular weight but did not have a heteroalkylene group in the main chain had greater minimum bactericidal concentrations and exhibited inferior disinfecting effects.

### Example 11

A 0.2% by weight aqueous solution of the cationic polymer prepared in Example 2 was prepared and the test of skin stimulation was conducted using the prepared solution.

### Example 12

The sterilizing disinfectant obtained in Example 8 was diluted and an aqueous solution containing 0.15% by weight of the cationic polymer prepared in Example 2 and 0.05% by weight of benzalkonium chloride was prepared. The test of skin stimulation was conducted using the prepared solution.

### Comparative Example 5

A 0.2% by weight aqueous solution of benzalkonium chloride was prepared and the test of skin stimulation was conducted using the prepared solution.

The results of Examples 11 and 12 and Comparative Example 5 are shown in Table 2.

**Table 2**

| | Male | | | | | Female | | | | |
|---|---|---|---|---|---|---|---|---|---|---|
| | A | B | C | D | E | F | G | H | I | J |
| Example 11 | | | | | | | | | | |
| 30 minutes | - | - | - | - | - | - | - | - | - | - |
| 24 hours | - | - | - | - | - | - | - | - | - | - |
| | | | | | | | | | | |
| Example 12 | | | | | | | | | | |
| 30 minutes | - | - | - | - | - | - | - | - | - | - |
| 24 hours | - | - | - | - | - | - | - | - | - | - |
| | | | | | | | | | | |
| Comparative Example 5 | | | | | | | | | | |
| 30 minutes | - | ± | - | - | ± | ± | ± | - | ± | ± |
| 24 hours | + | ++ | + | + | ++ | ++ | ++ | + | + | ++ |

The sterilizing disinfectants containing the cationic polymer represented by general formula [1] caused no skin disorder at all as shown by the result of Example 11. In contrast, benzalkonium chloride caused marked skin disorder as shown by the result of Comparative Example 5. Although the sterilizing disinfectant of Example 12 contained the cationic polymer represented by general formula [1] and benzalkonium chloride in amounts such that the ratio of the amounts was 1, 500: 500, this sterilizing disinfectant caused no skin disorder at all.

### INDUSTRIAL APPLICABILITY

Unlike benzalkonium chloride, the sterilizing disinfectant of the present invention causes no skin disorder even when the disinfectant is used in a concentration of 0.2% by weight which is the concentration in the ordinary use of sterilizing disinfectants. Moreover, the sterilizing disinfectant of the present invention exhibits a more excellent disinfecting effect than that of conventional sterilizing disinfectants.

## Claims

1. A sterilizing disinfectant comprising a cationic polymer represented by general formula [1]: 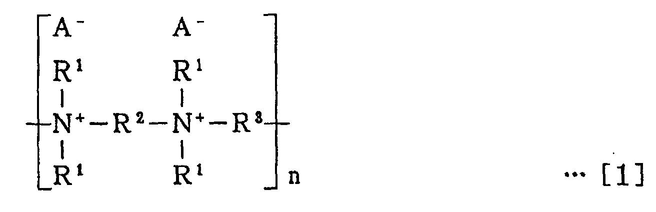 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500.

2. A sterilizing disinfectant according to Claim 1, wherein the cationic polymer has a weight-average molecular weight of 10,000 to 50,000.

3. A sterilizing disinfectant according to any one of Claims 1 and 2, which further comprises a sterilizing disinfectant based on a surfactant.

4. An antimicrobial agent comprising a cationic polymer represented by general formula [1]: 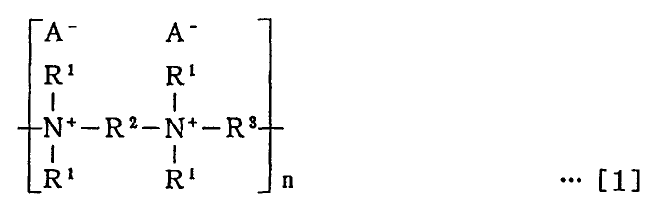 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500.

5. An antimicrobial material comprising a cationic polymer represented by general formula [1]: 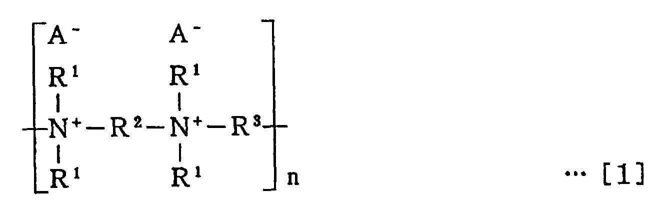 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500.

6. An antimicrobial material coated with a cationic polymer represented by general formula [1]: 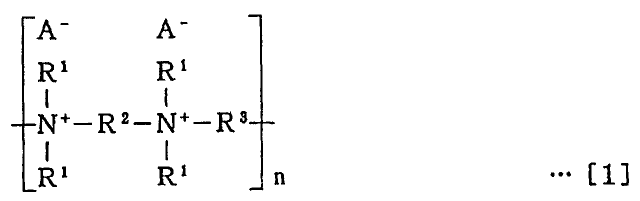 wherein R¹ represents an alkyl group having 1 to 4 carbon atoms, a hydroxyalkyl group having 1 to 4 carbon atoms or an alkenyl group having 2 to 4 carbon atoms, R² represents an alkylene group having 2 to 10 carbon atoms, R³ represents a heteroalkylene group having 2 to 6 carbon atoms or a heteroalkylene group having hydroxyl group and 2 to 6 carbon atoms, A⁻ represents an anion and n represents a number of 40 to 500.
